(19) Europäisches Patentamt
European Patent Office
Office européen des brevets

(11) Numéro de publication: **0 212 254 B1**

(12) # FASCICULE DE BREVET EUROPEEN

(45) Date de publication de fascicule du brevet: **09.10.91**

(51) Int. Cl.5: **C07C 45/34**, C07C 47/225, C07C 57/26, C07C 51/235, C07D 307/92

(21) Numéro de dépôt: **86109734.3**

(22) Date de dépôt: **16.07.86**

(54) **Procédé pour la préparation de composés oxygénés décaliniques.**

(30) Priorité: **12.08.85 CH 3445/85**

(43) Date de publication de la demande: **04.03.87 Bulletin 87/10**

(45) Mention de la délivrance du brevet: **09.10.91 Bulletin 91/41**

(84) Etats contractants désignés: **CH DE FR GB LI NL**

(56) Documents cités:
**GB-A- 701 911**

**HELVETICA CHIMICA ACTA, vol. 68, no. 212, fasc. 7, 13 novembre 1985, pages 2022-2029; G. OHLOFF et al.: "Significance of the geminal dimethyl group in the odor principle of Ambrox"**

**J. ORG. CHEM., vol. 44, no. 3, 1979, pages 456-458, American Chemical Society, D.V. RAO et al.: "Base-catalyzed autoxidation of cyclic ketones"**

(73) Titulaire: **FIRMENICH SA**
**1, route des Jeunes**
**CH-1211 Genève 8(CH)**

(72) Inventeur: **Naef, Ferdinand, Dr.**
**30, chemin Vert**
**CH-1227 Carouge(CH)**
Inventeur: **Vial, Christian**
**5, avenue du Lignon**
**CH-1219 Le Lignon(CH)**

(74) Mandataire: **Salvadori, Giuseppe, Dr.**
**c/o Firmenich S.A. Case Postale 239**
**CH-1211 Genève 8(CH)**

EP 0 212 254 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

L'AMBROX (marque enregistrée de Firmenich SA, Genève), ou 3a,6,6,9a-tétraméthylperhydronaphto-[2,1-b]furanne, constitue l'un des ingrédients de choix dans grand nombre de compositions parfumantes. Son odeur caractéristique d'ambre gris possède un pouvoir de diffusion particulier qui est resté à ce jour inégalé. Depuis sa découverte [voir Helv. Chim. Acta,33, 1251 (1950)], nombreuses ont été les synthèses proposées pour sa préparation par différents groupes de recherche. Celles-ci font généralement appel à la dégradation oxydative de diterpènes tel le sclaréol ou le manool, ou utilisent l'ambréine comme matériel de départ [G. Ohloff dans Fragrance Chemistry, ed. Ernst T. Theimer, p. 545, Academic Press (1982)].

Parmi les composés de structure apparentée à l'AMBROX décrit dans l'art antérieur figurent les aldéhydes oxygénés décaliniques de formule (III) possédant une liaison supplémentaire dans l'une des positions indiquées par les pointillés [voir brevets GB 701, 911 et DE-PS 1,019,031 et Parfümerie und Kosmetik, 54, 335 (1973)]. Très appréciés pour leur senteurs d'ambre gris, ces composés constituent en soi non seulement des ingrédients parfumants intéressants, mais également des intermédiaires utiles pour la préparation d'AMBROX.

En effet, les aldéhydes mentionnés ainsi que les acides correspondants peuvent être réduits en leurs carbinols correspondants, par exemple par traitement avec du $LiAlH_4$, lesquels composés, par cyclisation en milieu acide, fournissent l'AMBROX désiré selon le schéma réactionnel (a).

Les composés décaliniques oxygénés de formule (I) ont été obtenus dans le passé par des procédés (voir références indiquées plus haut) qui se sont révélés à l'expérience peu rentables, soit parce qu'ils ne permettaient pas d'atteindre des rendements intéressants, soit parce qu'ils faisaient intervenir des réactifs polluants ou avaient recours à des produits de départ difficilement accessibles.

Nous avons maintenant découvert qu'il était possible d'obtenir les composés (I), en particulier le composé possédant une double liaison exocyclique en position gamma, à l'aide d'un procédé original caractérisé en ce qu'on oxyde une cétone de formule (II) en milieu basique en présence d'oxygène. Il s'agit en l'espèce de l'application d'une réaction en soi connue, utilisée en particulier pour la transformation de cétones cycliques en leur dérivés acides dicarboxyliques [voir à ce sujet : D. V. Rao et al., J. Org. Chem. 44, 456 (1979) et T. S. Wallace et al., J. Org. Chem. 30, 3768 (1965)]. Le procédé de l'invention est caractérisé en ce qu'on oxyde la cétone de formule (II) à l'aide d'oxygène en un milieu basique constitué par du tert-butylate de potassium en solution dans le 1,2-diméthoxyéthane.

Le produit ainsi obtenu se présente sous forme d'un mélange contenant en parties à peu près équivalentes l'aldéhyde de formule (IV) et l'acide de formule (V).

Ce mélange est utilisé directement pour sa transformation successive en AMBROX suivant la méthode illustrée par le schéma réactionnel indiqué plus haut.

La réaction qui caractérise le procédé de l'invention s'effectue de préférence à une température contrôlée comprise entre environ 0° et +20°C. Suivant un mode d'exécution particulier, on ajoute la cétone de départ, dans une solution de 1,2-diméthoxyéthane, à un mélange refroidi de tert-butylate de potassium, également dans le 1,2-diméthoxyéthane, puis, lorsque le mélange obtenu a atteint une température d'environ +10°C, on introduit l'oxygène sous forme d'un courant continu ou en circuit pressurisé fermé. L'absorption d'oxygène est accompagnée par un dégagement de chaleur et la température du mélange réactionnel doit être maintenue dans les limites désirées (inférieur à 20°C) par un refroidissement extérieur.

Le mélange d'aldéhyde et acide désiré peut ensuite être séparé du mélange de réaction par extraction à l'aide d'un solvant organique, par exemple, au moyen d'éther diéthylique.

La cétone de formule (II) utilisée comme produit de départ dans le procédé de l'invention peut être obtenue conformément au procédé décrit par A. K. Dey et H. R. Wolf [Helv. Chim. Acta, 61, 1004 (1978)] à partir d'acide épéruique. Elle peut également être obtenue à partir de manool tel qu'il a été indiqué par E. Demole et H. Wuest [Helv. Chim. Acta, 50, 1314 (1967)].

L'invention est illustrée de manière plus détaillée par l'exemple suivant dans lequel les températures sont indiquées en degrés centigrades et les abréviations ont le sens usuel dans l'art.

### Exemple

Dans un ballon de 500 ml, équipé d'un septum, d'un thermomètre et d'un tube latéral permettant de placer l'appareillage sous atmosphère d'oxygène, on a introduit 150 ml de 1,2-diméthoxyéthane sec (filtré sur $Al_2O_3$ basique et conservé sur Linde 4 angströms) et 26,9 g (0,24 mole) de tert-butylate de potassium. On a refroidi à 5° (bain de glace), enclenché l'agitation magnétique et introduit à l'aide d'une seringue 15,72 g (0,06 mole) de 15,16-dinorlabd-8(20)-ène-13-one en solution dans 20 ml de 1,2-diméthoxyéthane

sec. Dès que la température est revenue vers 10°, on arrête l'agitation, on purge l'appareil à l'oxygène sec, on ferme hermétiquement tout l'appareillage, on ouvre l'alimentation d'oxygène (provenant d'une burette étalonnée) et on enclenche l'agitation. L'absorption d'oxygène commence aussitôt (exothermique) et après 25 min. De réaction (entre 10 et 16°, bain de glace), 2,75 litres d'oxygéne ont été absorbés et on note un net ralentissement de la réaction. On a enlevé le bain de glace, laissé remonter la température vers 20-25° et maintenu l'agitation encore 1 h 35. 1,55 litres d'oxygène supplémentaire ont été ainsi absorbés (soit au total 4,3 litres). On a versé le mélange de réaction sur de la glace, acidifié à HCl 10% glacé, extrait 2 fois à l'éther sulfurique, lavé jusqu'à neutralité avec une solution saturée de NaCl, séché sur $Na_2SO_4$ et concentré.

17,9 g de produit ont été obtenus dont un échantillon de 100 mg a été traité au diazométhane pour séparation à la chromatographie en phase gazeuse (SP 1000 5% sur Chromosorb W 80-100 mesh, 2,5 m, isoth. 240°).

On a pu ainsi déterminer que le produit obtenu était constitué par un mélange constitué à raison de 40% de 13,14,15,16-tétranorlabd-8(20)-ène-12-al[identique à un échantillon préparé conformément au procédé décrit dans la littérature (voir G. Ohloff, E. T. Theimer, ed. Academic Press, 1982, p. 535 et références citées)].

IR (film) : 3180, 2940, 2720, 1730, 1650, 1470, 1160, 905 $cm^{-1}$

$H^1$-RMN (60 MHz, $CDCl_3$) : 0,70 ; 0,82 ; 0,90 (3s,3H) ; 4,35 et 4,77 (2s larges, 2H) ; 9,55 (t : J = 2 Hz, 1H) delta ppm.

L'aldéhyde était accompagné de l'acide 13,14,15,16-tétranorlabd-8(20)-ène-12-oïque (à raison d'environ 45%), identifié par son ester méthylique qui possédait les caractères analytiques suivants :

IR (film) : 2950, 1745, 1650, 1470, 1440, 1170, 900 $cm^{-1}$

$H^1$-RMN (60 MHz, $CDCl_3$) : 0,70 ; 0,81 ; 0,89 (3s,3H) ; 3,60 (s,3H) ; 4,45 et 4,72 (2s larges, 2H) delta ppm.

La 15,16-dinorlabd-8(20)-ène-13-one, utilisée comme produit de départ dans le procédé décrit ci-dessus, a été préparée par oxydation permanganique du (+)-manool selon la méthode décrite par E. Demole et H. Wuest [Helv. Chim. Acta, 50, 1314 (1967)].

La transformation du mélange d'aldéhyde et acide obtenu en AMBROX s'effectue par réduction au moyen de $LiAlH_4$ en milieu d'éther diéthylique et cyclisation du carbinol résultant, ou 13,14,15,16-tétranorlabd-8(20)-ène-12-ol au moyen d'un agent de cyclisation acide.

## ANNEXE

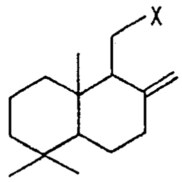

(I)

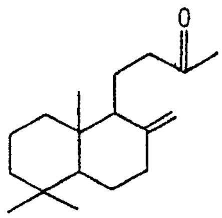

(II)

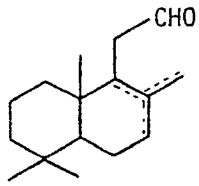

(III)

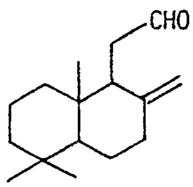

(IV)

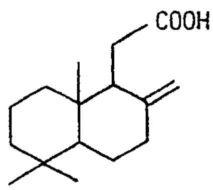

(V)

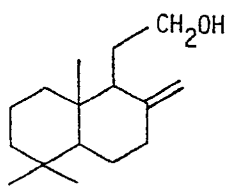

(VI)

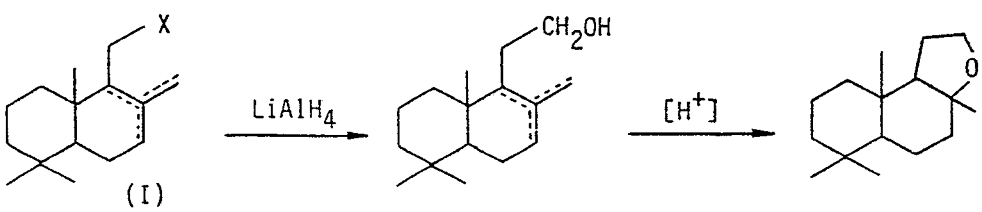


(I)

(a)

X = COOH
CHO

## Revendications

**1.** Procédé pour la préparation de composés oxygénés de formule

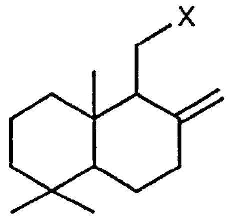

(I)

dans laquelle le symbole X représente un radical COOH ou CHO, caractérisé en ce qu'on oxyde une cétone de formule

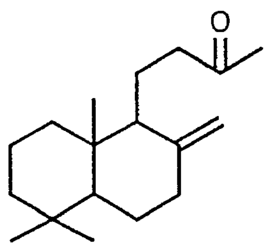

(II)

à l'aide d'oxygène en un milieu basique constitué par du tert-butylate de potassium en solution dans le 1,2-diméthoxyéthane.

**2.** Procédé selon la revendication 1, caractérisé en ce que la réaction s'effectue à une température comprise entre environ 0° et +20° C.

**3.** Procédé selon la revendication 1, caractérisé en ce que la réaction s'effectue à pression atmosphérique ou à une pression légèrement supérieure à celle-ci.

**4.** Utilisation du produit obtenu selon le procédé de la revendication 1 à titre de produit de départ pour la préparation de 3a,6,6,9a-tétraméthylperhydronaphto[2,1-b]furanne par réduction dudit produit en carbinol de formule

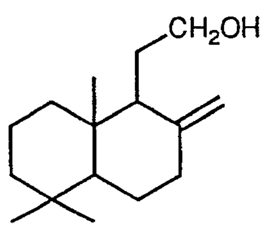

(VI)

à l'aide d'un hydrure métallique et cyclisation du carbinol obtenu au moyen d'un réactif de cyclisation acide.

**Claims**

**1.** Process for the preparation of oxygen containing compounds of formula

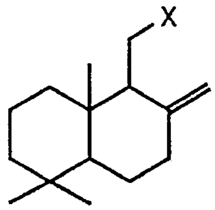

(I)

where symbol X represents a COOH or CHO radical, characterized in that a ketone of fomula

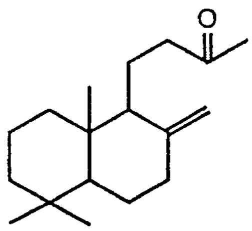

(II)

is oxidized by means of oxygen in a basic medium consisting of potassium tert-butoxide in solution in 1,2-dimethoxyethane.

2. Process according to claim 1, characterized in that the reaction is carried out at a temperature of between about 0° and +20° C.

3. Process according to claim 1, characterized in that the reaction is carried out at atmospheric pressure or at a pressure slightly higher than it.

4. Utilization of the product obtained according to the process of claim 1 as raw material for the preparation of 3a,6,6,9a-tetramethylperhydronaphtho[2,1-b]furan by reduction of the said product by means of a metal hydride to give a carbinol of formula

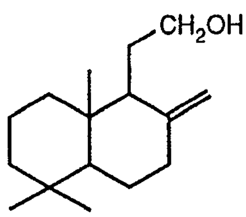

(VI)

and cyclization of the obtained carbinol by means of an acidic cyclization reagent.

**Patentansprüche**

1. Verfahren zur Herstellung von Sauerstoff-haltigen Verbindungen der Formel

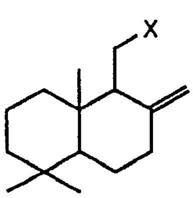

(I)

worin das Symbol X ein COOH oder ein CHO Radikal darstellt, dadurch gekennzeichnet, daß man ein Keton der Formel

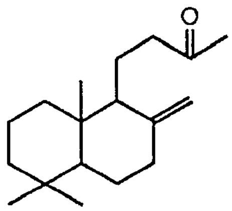

(II)

mittels Sauerstoff in einem basischen Milieu von Kalium tert-Butoxyde in einer 1,2-Dimethoxyethane Lösung oxydiert.

2. Verfahren gemäß Anspruch 1, dadurch gekennzeichnet, daß die Reaktion bei einer Temperatur zwischen ca. 0° und 20° C durchgeführt wird.

3. Verfahren gemäß Anspruch 1, dadurch gekennzeichnet, daß die Reaktion bei normalem Druck oder bei leicht erhöhtem Druck durchgeführt wird.

4. Verwendung von dem Produkt, das gemäß des Verfahrens von Anspruch 1 hergestellt wurde, als Ausgangsmaterial zur Herstellung von 3a,6,6,9a-Tetramethylperhydronaphtho[2,1-b]furan durch Reduktion des genannten Produktes mittels eines Metall Hydrids, um einen Carbinol der Formel

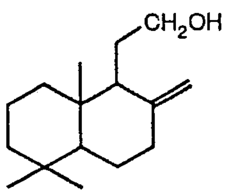

(VI)

zu liefern, und die Cyclisierung des so erhaltenen Carbinols mittels eines sauren Cyclisierungsreagenzes.